# EUROPEAN PATENT APPLICATION

(11) **EP 1 632 137 A1**
(43) Date of publication of application: **08.03.2006**
(21) Application number: 05107061.3
(22) Date of filing: 29.07.2005
(51) Int. Cl.: A23L 1/30, A61K 9/14, A61K 31/575, A61K 47/36, A61K 47/42, A23L 1/09, A23L 1/29, A61J 3/02

(54) **Compositions and processes for water-dispersible phytosterols and phytostanols**

(30) Priority: 10.08.2004 US 915147
(71) Applicant: KRAFT FOODS HOLDINGS, INC., Northfield, Illinois 60093 (US)
(72) Inventor: Gaonkar, Anilkumar Ganapati, 60089, Buffalo Grove (US); McPherson, Andrew, Mount Prospect, Illinois 60056 (US); Topinka, John B., Des Plaines, Illinois 60016 (US); Finley, John Westcott, Lansdale, Pennsylvania 19446 (US)
(74) Representative: Smaggasgale, Gillian Helen

(57) **Abstract**

Plant and plant sterol esters have been shown to be cholesterol-reducing agents in human serum. In the present invention, plant sterols and plant stanols are incorporated into compositions which are dispersible in water by complexing the hydrophobic lipids with water-soluble carbohydrates. The resulting plant sterol-carbohydrate complexes are in an aqueous or powder form which can be readily incorporated with food products in an amount effective to reduce serum cholesterol levels in a human consuming such food product, without adversely modifying the organoleptic properties of the food product.

## Description

### FIELD OF THE INVENTION

The present invention relates to formulations and processes for incorporating plant sterols and/or plant stanols into food products. Specifically, the plant sterols and plant stanols described herein are complexed with digestible or partially digestible carbohydrates such that they are advantageously water dispersible and may be easily incorporated into water-based foods. The plant sterol-carbohydrate complexes and/or plant stanol-carbohydrate complexes of the present invention may be produced in a powder form by way of extrusion in the presence of a carbohydrate (e.g., starch, maltodextrin, hydrocolloids, grain-based flours, and the like) or by drying the plant sterol-carbohydrate complexes and/or plant stanol-carbohydrate complexes using conventional techniques. The plant sterol-carbohydrate complexes and/or plant stanol-carbohydrate complexes can deliver relatively high levels of the cholesterol-reducing agents without an adverse effect on the food product to which it was incorporated.

### BACKGROUND OF THE INVENTION

There remains considerable interest in reducing cholesterol levels. Cholesterol absorbed from dietary sources can increase overall cholesterol levels. Other than avoidance or reduced consumption of high cholesterol foods, measures available without prescription to the general public to reduce the absorption of cholesterol from the diet have met with little success. Furthermore, in many cases, high serum cholesterol cannot be significantly reduced by lowering dietary cholesterol. However, high cholesterol levels in serum can be lowered effectively by altering the intestinal metabolism of lipids. In recent years, it has become known that certain plant sterols and plant stanols such as β-sitosterot (24-ethyl-5-cholestene-3β-ol) and its hydrogenated form β-sitostanol (24-ethyl-5α-cholestane-3β-ol) can help lower serum cholesterol by inhibiting cholesterol absorption in the digestive system. Other examples of plant sterols include campesterol, stigmasterol, and ergosterol. Plant stanols are the hydrogenated form of plant sterols. Other examples of plant stanols include campestanol. *See, e.g.,* "Reduction of Serum Cholesterol With Sitostanol-ester Margarine in a Mildly Hypercholesterolemic Population", *New Engl. J. Med*., 333, 1308-1312 (1995); "Short-Term Administration of Tall Oil Phytosterols Improves Plasma Lipid Profiles in Subjects with Different Cholesterol Levels," *Metabolism: Clinical & Experimental*, 47, 751-756 (1998); and "Cholesterol-lowering Efficiency of a Sitostanol-containing Phytosterol Mixture with a Prudent Diet in Hyperlipidemic Men," *Am. J. Clin. Nutr*., 69, 1144-50 (1999).

The use of plant sterols, which are natural components of vegetable fats and oils, in food products is considered safe. Plant sterols themselves are not absorbed, or only absorbed in very small amounts, from the intestines. A decreased incidence of coronary disease is clearly associated with a decrease in serum cholesterol and, in particular, a decrease in LDL cholesterol. A high serum cholesterol level is one of the most significant indicators of coronary disease. There are a wide variety of naturally occurring plant sterols which have been reported to have a cholesterol-reducing effect, although not all have equivalent action.

Although the mechanism by which plant sterols achieve the effect of lowering serum cholesterol has not been fully elucidated, and not wishing to be limited to theory, it is generally believed that plant sterols interfere with cholesterol absorption by competition-type mechanisms. Cholesterol absorption appears to take place primarily in the proximal third of the small intestine. Cholesterol esters must be converted to their free hydroxyl form by the action of cholesterol esterases before they can be absorbed. The free cholesterol requires bile salts for solubilization and absorption. Bile salts form an aqueous dispersion of micelles in which the cholesterol is solubilized along with phospholipids and hydrolysis products of other dietary lipids. Micelles transport the cholesterol across the hydrophilic barrier (the unstirred water layer) to reach the surface of the intestinal mucosa. At the mucosa, it is thought that the cholesterol dissociates from the micelle and is transported into the mucosa cells by a process which has not yet been fully defined but may include passive exchange diffusion or by protein-mediated transport. Plant sterols could interfere with cholesterol absorption by the following general mechanisms: (a) competition with cholesterol for absorption into the bile-salt micelles and/or (b) competition with the transport mechanism into the mucosa cells.

Conventionally, plant sterols have been incorporated into food products by melting a sterol or stanol, incorporating it into an oil phase, and blending the oil phase with other components to result in a plant sterol-containing food product. However, plant sterols have high melting points (i.e., about 100 to 180°C) which can result in the crystallization of the plant sterols within the oil phase of such food products. Such crystallization of the plant sterols within the oil phase results in a gritty and unacceptable texture. This gritty texture is especially detectable when the oil/plant sterol phase is incorporated at high levels in the food products. The high melting points and hydrophobic nature of such plant sterols also makes it difficult to blend such plant sterols with an aqueous phase. Furthermore, actual melting of the plant sterol for incorporation into food products is energy intensive.

Attempts have been made to solve these problems using, for example, chemical modification of the plant sterols. For example, esterification of plant sterols generally results in lowered melting temperatures, which leads to increased solubility of the cholesterol reducing agent. Thus, such plant sterol esters generally may be incorporated into food products more readily due to lower melting points and can provide food products without significantly gritty texture. Additionally, esterification of plant sterols may result in the increased bioavailability. See, *e.g*., U.S. Patent 5,502,045 and European Patent Application WO 99/59421.

In another example, European Patent WO 99/56729 describes food compositions wherein a melt and/or solution of high melting phytosterols in monomolecular, low associated, or "cluster" forms are immobilized and stabilized in a matrix. This method reportably results in a better homogenous incorporation of high-melting phytosterols in comparison to other methods.

It would be desireable, therefore, to provide additional forms of plant sterols which can be incorporated into food products at relatively high levels without adversely affecting texture or other organoleptic properties. It would also be desirable to provide forms of plant sterols which can more easily be incorporated into food products. It would also be desirable to provide forms of plant sterols which can more easily be incorporated into food products without requiring significant modification of existing food production lines or methods. It would also be desirable to provide methods for preparing such plant sterols without chemical modification of the plant sterol itself. It would also be desirable to provide forms of plant sterols in aqueous dispersions. The present invention provides such plant sterols and methods for preparing them.

### SUMMARY OF THE INVENTION

The present invention relates to methods and compositions of modified plant sterols for incorporation into a food product. The plant sterols described herein are complexed with a digestible or partially digestible carbohydrate (e.g., starch, maltodextrin, hydrocolloid, grain-based flour, and the like) such that the resultant product may be directly incorporated into a food product in an amount effective to reduce serum cholesterol levels in a human consuming the food products without adversely affecting the organoleptic properties of the food product.

The plant sterol-carbohydrate complexes described herein are advantageously water soluble such that the complexes may be incorporated into water based foods. Additionally, the sterol-carbohydrate complexes may be extruded in the presence of a carbohydrate (e.g., starch, maltodextrin, hydrocolloid, grain-based flour, and the like) such that the resultant complex is in a powder form which is readily dispersable in all different types of food products. Alternatively, the plant sterol-carbohydrate complexes may be dried (e.g., spray drying or freeze drying) prior to use if desired.

The plant sterol-carbohydrate complexes described herein may include sterols, stanols, or the esters of either sterols or stanols. Any carbohydrate may be complexed with a plant sterol, but preferably a complex carbohydrate (e.g., amylose-containing starch, hydroxypropylated starch, acetylated starch, octenyl succinylated (OSA) starch, and the like) is used. Crystal inhibitors may be added to the plant sterol-carbohydrate complexes in order to further inhibit crystal growth of the sterols.

### DETAILED DESCRIPTION

The present invention provides food products such as, for example, full-fat, low-fat, fat-free, and triglyceride-free foods, which incorporate plant sterols as cholesterol-reducing compounds. The cholesterol-reducing compounds are incorporated into food products by adding them as plant sterol-carbohydrate complexes, including sterol-carbohydrate and/or stanol-carbohydrate complexes. The sterol-carbohydrate and/or stanol-carbohydrate complexes descried herein may be dispersibly added to aqueous solutions. Furthermore, the sterol-carbohydrate and/or stanol-carbohydrate complexes described herein may be further processed into a powder form by using an extruder or other drying methods (e.g., spray drying, freeze drying, and the like).

For purposes of this invention, "plant sterols" is intended to include both plant sterols and plant stanols; the term "sterols" alone is intended to include only the plant sterols; and the term "stanols" alone is intended to include only the plant stanols. For purposes of this disclosure, a "cholesterol-reducing-compound" is defined as a plant sterol as defined herein. The plant sterols used in this invention may be obtained from any conventional source, including, for example, commercially available plant sterols, plant sterols derived from appropriate plant materials, plant sterols derived as a deodorized distillate from vegetable oil processing, and the like.

The plant sterol-carbohydrate complexes, whether aqueous or powder based, can be incorporated into full-fat, low-fat, fat-free, and triglyceride-free food products in a cholesterol-reducing amount without imparting gritty texture or other undesirable organoleptic properties to the resulting food products. Such food products include, but are not limited to, pourable dressings, spoonable dressings, drinks, confections, ice creams, whipped desserts, whipped toppings, frozen dairy foods, dairy products (e.g., milk, yogurt, cheese, cream cheese, process cheese, creamers, and the like), dips, sauces, soups, desserts, dessert toppings, chocolate products, spreads, sour cream products, cream cheese products, processed meats, cereals, and the like; such food products may be in the form of a ready made product or a product which the consumer prepares (e.g., a dry dressing mix to which the consumer adds, as desired, water and/or oil). The use of such plant sterol-carbohydrate complexes allows the incorporation of the plant sterol in food products at a relatively high level without producing a gritty texture. Moreover, because these complexes may be water-based and/or in powder form, they provide great flexibility in the manufacturing process. For example, the powder form and aqueous form of the plant sterol-carbohydrate complex may be used to produce low-fat or fat-free food products. In most cases, such plant sterol-carbohydrate complexes can be added to the desired food product with only minimal modification to existing food production lines; the powder form is particularly advantageous in this regard. For example, powdered plant sterol-carbohydrate complexes may be sprinkled on the top of any food in order to deliver a cholesterol-reducing amount of the complex without adversely affecting the organoleptic properties of the food product to which it was added. Although the plant sterol-carbohydrate complexes described herein are mainly intended to be used in food products, they can, of course also be used in pharmaceutical and dietary supplement applications for their cholesterol-reducing effect. In addition to being simply added to the appropriate food products, they can be provided in a sachet for use in products such as powdered drink mixes, dry salad dressing mixes, non-dairy creamers or whiteners, and the like. They can also be sprinkled on various foods (e.g., salads, soups) to provide the benefits of sterols.

For purposes of this invention, the term "low-fat" includes "reduced-fat," "light," "low-fat," and "fat-free" as defined by the FDA Standards of Identi In addition, for purposes of this invention, "low-fat" also encompasses triglyceride-free products although they are not included in the FDA Standards of Identity. In addition, the terms "low-fat" and "fat-free" are intended to include low-fat and fat-free food products that do not fall into the categories defined by the FDA Standards of Identity (as of the time of this disclosure) but deliver to the consumer reduced levels of fat per serving. Furthermore, through the use of higher levels of plant sterol esters, even if no oil is added in a formulation of the described compositions, while such a composition may not be termed "fat-free", the term "triglyceride-free" is an accurate description of such formulations utilized in this disclosure.

Generally, plant sterol-carbohydrate complexes may be formed by melting a carbohydrate and a sterol/stanol in a mixture with water. The gelatinization temperature of carbohydrates is generally above about 60°C depending on the specific carbohydrate (e.g., about 65°C for corn starch to about 120°C for high-amylose starch; see Zobel, In Starch: Chemistry and Technology (Eds. Whistler, R.L., BeMiller, J.N., and Paschall, E.F.), 2nd Ed., Academic Press, New York, pp 285-310, 1984)), whereas the melting temperature of most plant sterols is between about 130 and 180°C. By complexing the hydrophobic plant sterol with a water-soluble carbohydrate, the resulting plant sterol-carbohyd rate complex is water dispersible and/or water soluble. Advantageously, the resultant water-dispersible plant sterols can be homogenously dispersed in an aqueous solution and are less susceptible to plant sterol crystal formation than other types of plant sterol dispersions, such as in oil dispersions. In another embodiment, a crystal growth inhibitor such as, for example, polyglycerol ester (such as PGE 55AK from Danisco, New Century, KS) and polyglycerol polyricinoleate (PGPR) can be added to the mixture to further inhibit the formation of sterol crystals.

Generally, the plant sterol-carbohydrate complexes of this invention contain the plant sterol and carbohydrate in a weight ratio of about 0.25:99.75 to about 90:10, preferably about 10:90 to about 90:10, and most preferably about 40:60 to about 60:40. When a crystal inhibitor is used, the plant sterol-carbohydrate-crystal inhibitor complexes of this invention contain the plant sterol, carbohydrate, and crystal inhibitor in a weight ratio of about 0.25:99.7:0.05 to about 85:10:5, preferably about 9:89:2 to about 90:9.5:0.5, and most preferably about 49:49:2. The crystal inhibitor retards the sterol/stanol crystal growth (initiation and propagation) so that grittiness is not perceived in the product. Preferably, the crystal inhibitor is used in the complex. Although the crystal inhibitor is not readily soluble in water, it can easily be mixed with the sterol/stanol at the higher temperature (above its melting temperature) normally used in the extruder.

The plant sterol-carbohydrate complex can, if desired, be obtained in a solid form. For example, a plant sterol, carbohydrate, and water mixture may be extruded at about 80 to about 200°C, preferably at about 150 to about 170°C, in the presence of a carbohydrate (e.g., starch, maltodextrin, and the like) to form a powdered plant sterol-carbohydrate complex. Using this extrusion technique, the carbohydrate (e.g., starch and/or maltodextrins) is preferably dry blended with the sterol and/or stanol and an optional crystal inhibitor with sufficient mixing to form a homogeneous dispersion of the sterol and/or stenol and optional crystal inhibitor in the carbohydrate. The dry blend is preferably fed into the extruder wherein water (generally at least about 25 percent and preferalby about 25 to about 50 percent of the weight of the dry blend) is injected into the extruder barrel to mix with the dry blend. The resulting mixture is then extruded at a temperature sufficient to melt the sterol and/or stanol and effect gelatinization of the carbohydrate and form a homogenous plastic mass in the extruder barrel. An extrusion temperature should be higher than melting point (and preferably at least about 10°C above the melting point) of the plant sterol. Generally, the extrusion temperature should be below the caramalization temperature of the carbohydrate. Preferably, extrusion is carried out under low shear conditions (e.g., low speeds and low die pressure) in order to reduce or eliminate shear damage to the carbohydrate. A majority of the water is flashed off as the extrudate exits the extrusion barrel and the extrudate forms a structurally stable shape depending on the extrusion die. The extrudate is then dried to a moisture content of about 5 to about 15 percent moisture; preferably, the extrudate is allowed to air dry. The resulting extrudate is generally white to off-white and, depending extrusion conditions can be relatively dense to highly expanded. Alternatively, other drying methods (e.g., spray drying, freeze drying, and the like) can be used to dry the form once the complex is formed.

Preferred carbohydrates for use in this invention include amylose-containing starches (more preferably octenyl succinylated starches), acetylated starches, and/or acid-thinned starches. Generally, as the amylose content of starch increases, its ability to form complexes with hydrophobic molecules, such as fatty acids, increases. See, *e*.*g*., A. Young, "Fractionation of Starch" *in* Starch: Chemistry and Technology, 2^{nd} Ed, pp 249-283, 1984 (R. Whistler, J. BeMiller, and E. Paschall, eds). The preferred octenyl succinylated (OSA) starches (e.g., N-Creamer 46 from National Starch and Chemical, Bridgewater, NJ) can also serve as surface active and/or thickening agents; they have also been used as encapsulating agents, beverage emulsifiers, viscous emulsifiers, and dry flow agents. *See, e*.*g*., P. Trubiano, "Succinate and Substituted Succinate Derivatives of Starch" *in* Modified Starches: Properties and Uses, pp 131-148, 1986 (O. Wurzburg, ed). Although not wishing to be limited by theory, it appears that the linear amylose substituents in amylose starches, octenyl secinylated starches, or acetyl substituted starches can complex with the free sterols in different ways. First, amylose can form a helical inclusion complex with a portion of the sterol or stanols. Such inclusion complexes are generally very stable; such stability may contribute to the retardation or elimination of sterol/stanol crystal growth in the sterol-carbohydrate and stanol-carbohydrate complexes of this invention. Secondly, the OSA/acetyl substituents could complex via the helical inclusion mechanism as well as the traditional surface active (i.e., emulsifier) mechanism.

In yet another embodiment, plant sterols may be complexed with certain proteins to form a water-dispersible plant sterol-protein complex. Examples of such proteins include zein, wheat gluten and its fractions (e.g., gliadin, glutenin), wheat flour, wheat protein concentrate, soy flour, soy protein concentrate, and soy protein isolates. Complexes containing both carbohyrates and proteins can be prepared. Generally, the same methods are used to form plant sterol-protein complexes as described herein for the plant sterol-carbohydrate complexes. However, most proteins denature at temperatures lower than the melting point of plant sterols; therefore, the preferred embodiment is to complex plant sterols with carbohydrates.

Sterol-carbohydrate complexes and stanol-carbohydrate complexes may be formed by heating a mixture of a sterol or a stanol with a carbohydrate such as, for example, high-amylose starch, maltodextrin, corn starch, and octenyl succinylated (OSA) or acetylated starch, in water at a temperature between about 100 and about 190°C, preferably about 150 to about 170°C, under a pressure sufficient to keep the water from boiling out or flashing off, for a period of time between about 15 minutes and about 2 hours, preferably about 1 hour, and preferably with stirring. Stirring can be achieved by any conventional means. As noted, the process should be carried out under a pressure sufficient to prevent the water from boiling out as the mixture is heated. As will be appreciated by one of ordinary skill in the art, the temperature used in the processes described herein may vary depending on the melting point of the sterol or stanol used. Likewise, the process may take more or less than an hour depending on certain variable such as, for example, the sterol or stanol used, the carbohydrate used, and the amount of water added. In another embodiment, the sterol-carbohydrate complex or the stanol-carbohydrate complex may be extruded which results in a final product in the powder form.

The following examples are intended to further describe and not to limit the invention. All percentages and ratios used in the present specification are by weight, unless otherwise noted. All references cited herein are incorporated by reference.

### Example 1. Production of a Sterol-Starch Complex.

**Run 1.** 100g sitosterol-rich sterol from Cargill, 50g Hylon VII starch (High-amylose unmodified corn starch from National Starch and Chemical, Bridgewater, NJ), and 150ml water were mixed in a high pressure Brabender visco amylograph. The mixture was heated to 150°C for 30 minutes at a pressure (about 15 psi) sufficient to prevent the water from boiling off. The resulting product was not entirely melted; however, differential scanning calorimetry (DSC) data showed that a sterol-carbohydrate complex was formed.

**Run 2.** 1 part sitosterol-rich sterol from Cargill, 1.5 parts Hylon VII, and 5 parts water were added to a Parr Bomb at 170°C for 30 minutes; the system was closed and, therefore pressurized. The resulting product was brownish in color and was readily dispersible in water. In further testing, the aqueous solution was mixed with an equal amount of oil and allowed to stand for 1 day. Micrographs of the aqueous/oil solution after 1 day showed that the solution was essentially free of sterol crystals.

### Example 2. Incorporation of Crystal Growth Inhibitors to Plant Sterol Solutions.

**Run 1.** A mixture of 10g water, 5g sitosterol-rich sterol from Cargill, 5g Hylon VII, and 0.6g polyglycerol ester (PGE 55AK from Danisco, New Century, KS) was formed and heated to 160°C under pressure in a Parr Bomb for about 1 hour and slowly cooled to ambient temperature. A micrograph of the complex after cooling revealed only slight crystal formation (although fewer crystals were present than expected in a solution without the addition of a crystal inhibitor).

**Run 2**. A mixture of 10g water, 5g sitosterol-rich sterol from Cargill, 5g Hylon VII, and 0.6g polyglycerol ester (PGE 55AK) was formed and heated to 160°C under pressure in a Parr Bomb for about 1 hour and rapidly cooled to ambient temperature. A micrograph of the complex after cooling revealed smaller crystals than those formed in Run 1.

**Run 3.** A mixture of 10g water, 5g sitosterol-rich sterol from Cargill, 5g Hylon VII, and 0.6g polyglycerol polyricinoleate (PGPR 90 from Danisco, New Century, KS) was formed and heated to 160°C under pressure and slowly cooled to ambient temperature. A micrograph of the complex after cooling revealed slight crystal formation (although fewer crystals were present than expected in a solution without the addition of a crystal inhibitor).

**Run 4.** A mixture of 10g water, 5g sitosterol-rich sterol from Cargill, 5g Hylon VII, and 0.6g PGPR 90 was formed and heated to 160°C in a Parr Bomb for about 1 hour and rapidly cooled to ambient temperature. A micrograph of the complex after cooling revealed smaller crystals than those formed in Run 3.

**Example 3.** This example illustrates the preparation of sterol-starch complexes using extrusion. Such plant sterol-carbohydrate complexes may be formed by extruding a carbohydrate and a sterol or stanol (or the mixture of sterol and stenol) in a mixture with water. By complexing the hydrophobic plant sterol with a water-soluble carbohydrate, the resulting plant sterol-carbohydrate complex is water-dispersible and/or water-soluble. A crystal growth inhibitor such as, for example, polyglycerol ester (such as PGE 55AK from Danisco) and polyglycerol polyricinoleate (such as PGPR 90 from Danisco) can be added to the mixture to further inhibit the formation of sterol crystals.

**Run 1.** Preparation of a Sterol-Corn Starch (Melogel) Complex at a 50:50 Weight Ratio. Corn starch (1136g; Melogel from National Strach and Chemical, Bridgewater, NJ; moisture content = 13.6%) was dry blended with 1000g of sterol with sufficient mixing to form a homogeneous dispersion of sterol and starch at 50:50 ratio on dry starch basis. The dry blend was fed into a twin screw extruder (Werner Pfleiderer Corporation, Ramsey, NJ) wherein water (about 30 percent of the dry blend) was injected into the extruder barrel to mix with the dry blend. The resulting mixture was extruded to a maximum barrel temperature of about 150°C. This temperature is sufficient to melt the sterol and/or stanol and effect gelatinization of the corn starch and form a homogenous plastic mass in the extruder barrel. A majority of the water was flashed off as the extrudate exits the extrusion barrel; the extrudate forms a structurally stable shape. The extrudate was air dried to a moisture content of about 7 percent. The resulting extrudate was off-white in color.

**Run 2.** Preparation of a Sterol-OSA Starch Complex at a 50:50 Ratio. OSA starch (2272g; (N-Creamer 46 from National Starch and Chemical; moisture content = 13.6%) was dry blended with 2000g sterol with sufficient mixing to form a homogeneous dispersion of sterol and OSA starch at a 50:50 ratio on dry OSA starch basis. The dry blend was fed into the twin screw extruder used in Run 1 wherein water (about 30 percent of the dry blend) was injected into the extruder barrel to mix with the dry blend. Extrusion was carried out as in Run 1. The extrudate was air dried to a moisture content of 7.4 percent. The resulting extrudate was off-white in color.

**Run 3.** Preparation of a Sterol-OSA Starch-PGE 55AK Complex at a 49:49:2 Ratio: OSA starch (1113g) as in Run 2 was dry blended with 980g of sterol and 40g of a polyglycerol ester (PGA 55AK) crystal growth inhibitor with sufficient mixing to form a homogeneous dispersion. The dry blend was fed into the twin screw extruder used in Run 1 wherein water (about 30 percent of the weight of the dry blend) was injected into the extruder barrel to mix with the dry blend. Extrusion was carried out as in Run 1. The extrudate was then air dried to a moisture content of 7.3 percent. The resulting extrudate was off-white in color.

### Example 4. Incorporation of a Sterol-Starch Complex into Creamy Italian Dressing.

**Control Run.** Production of Creamy Italian Dressing without the addition of a Sterol-Starch Complex. As a control, creamy Italian Dressing was produced without the addition of sterol-corn starch complex according to the following formula:

| **Ingredient** | **Amount (%)** |
|---|---|
| Water | 62.9 |
| Soybean Oil | 14.0 |
| Vinegar, 120 grain | 7.7 |
| Spices/Flavorings | 4.9 |
| Sugar | 4.2 |
| Garlic Puree in Vinegar | 4.0 |
| Salt | 1.8 |
| Xanthan Gum | 0.4 |
| Potassium Sorbate | 0.05 |
| Vitamin E | 0.006 |
| EDTA | 0.006 |

Water, sugar, salt, vitamin E, EDTA, and potassium sorbate were added to a Hobart bowl in the amounts indicated above. The ingredients were then mixed at a first speed until the sugar and salt dissolved. Next, the mixing speed was increased to a second speed and mixing continued for an additional 2 minutes. Then, the mixing speed was reduced to the first speed and a slurry of xanthan gum in a part of the oil specified above was added to the mixture. The remaining soybean oil was added. The blend was then mixed for an additional 2 minutes. Then, the vinegar was added followed by mixing for 1 minute. The blend was then homogenized by passing it through a Hydroshear homogenizer. Next, spices and flavorings (including garlic puree) were added according to the above formula and the entire blend was mixed for 1 minute.

As expected, the resultant product exhibited the normal qualities of creamy Italian dressing. Further testing showed that the percent acid of the product was 1.16%, the pH was 3.54, and viscosity was 2000 cp at room temperature (Brookfield Model DV-II+ Viscometer; Spindle #4; rpm =20).

**Run 1.** Incorporation of 6.5% Sterol-Corn Starch (Melogel) Complex in a 50:50 ratio into Creamy Italian Dressing. The complex was prepared using a twin screw extruder using the procedure described in Example 3 (Run 1).

In this run 1, creamy Italian dressing was produced with the addition of the 6.5% sterol-corn starch complex at a 50:50 ratio according to the formula below:

| **Ingredient** | **Amount (%)** |
|---|---|
| Water | 56.4 |
| Soybean Oil | 14.0 |
| Vinegar, 120 grain | 7.7 |
| Sterol/Corn Starch Complex (50:50 Ratio) | 6.5 |
| Spices/Flavorings | 4.9 |
| Sugar | 4.2 |
| Garlic Puree in Vinegar | 4.0 |
| Salt | 1.8 |
| Xanthan Gum | 0.4 |
| Potassium Sorbate | 0.05 |
| Vitamin E | 0.006 |
| EDTA | 0.006 |

Water, sugar, salt, vitamin E, EDTA, and potassium sorbate were added to a Hobart bowl according to the formula shown above. The ingredients were then mixed at a first speed until the sugar and salt dissolved. Next, the mixing speed was increased to a second speed and extruded sterol-corn starch complex (in a 50:50 ratio) was added to the blend according to the formula shown above; mixing continued for an additional 2 minutes. Then, the mixing speed was reduced to the first speed and a slurry of xanthan gum in a part of the oil specified above was added to the mixture. The remaining soybean oil was added. The blend was then mixed for an additional 2 minutes. Then, the vinegar was added followed by mixing for 1 The blend was then homogenized by passing it through a Hydroshear homogenizer. Next, spices and flavorings (including the garlic puree) were added according to the above formula and the entire blend was mixed for 1 minute.

The resultant product was similar to the control dressing in taste. Additionally, the dressing from run 1 did not exhibit any gritty and/or grainy mouthfeel generally associated with free sterol. Further testing showed that the percent acid of the product was 1.09%, the pH was 3.55, and viscosity was 3360 cp at room temperature. A single serving of the dressing (about 31 g) would provide about 1 g sterol.

**Run 2.** Incorporation of 6.5% Sterol-OSA Starch Complex in a 50:50 ratio into Creamy Italian Dressing. In run 2, creamy Italian dressing was produced with the addition of 6.5% sterol-octeyl succinic starch complex (prepared in a manner similar to that described in Example 3 (Run 2)) according to the formula below:

| **Ingredient** | **Amount (%)** |
|---|---|
| Water | 56.4 |
| Soybean Oil | 14.0 |
| Vinegar, 120 grain | 7.7 |
| Sterol/OSA Starch Complex (50:50 Ratio) | 6.5 |
| Spices/Flavorings | 4.9 |
| Sugar | 4.2 |
| Garlic Puree in Vinegar | 4.0 |
| Salt | 1.8 |
| Xanthan Gum | 0.4 |
| Potassium Sorbate | 0.05 |
| Vitamin E | 0.006 |
| EDTA | 0.006 |

Water, sugar, salt, vitamin E, EDTA, and potassium sorbate were added to a Hobart bowl according to the formula shown above. The ingredients were then mixed at a first speed until the sugar and salt dissolved. Next, the mixing speed was increased to a second speed and extruded sterol-OSA starch complex in a 50:50 ratio was added to the blend according to the formula shown above; mixing continued for an additional 2 minutes. Then, the mixing speed was reduced to the first speed and a slurry of xanthan gum in a part of the oil specified above was added to the mixture. The remaining soybean oil was added. The blend was then mixed for an additional 2 minutes. Then, the vinegar was added followed by mixing for 1 minute. The blend was then homogenized by passing it through a Hydroshear homogenizer. Next, spices and flavorings (including garlic puree) were added according to the above formula and the entire blend was mixed for 1 minute.

The resultant product was similar to the control dressing in taste. Additionally, the dressing from run 2 did not exhibit any gritty and/or grainy mouthfeel generally associated with free sterol. Further testing showed that the percent acid of the product was 1.23%, the pH was 3.65, and viscosity was 1040 cp at room temperature. A single serving of the dressing (about 31 g) would provide about 1 g sterol.

**Run 3.** Incorporation of 6.5% Sterol-OSA Starch-PGE 55AK Complex at a 49:49:2 ratio into Creamy Italian Dressing. In run 3, creamy Italian dressing was produced with the addition of 6.5% sterol-OSA starch-PGE 55AK complex at a 49:49:2 ratio (prepared in a manner similar to that described in Example 3 (Run 3)) according to the formula below:

| **Ingredient** | **Amount (%)** |
|---|---|
| Water | 56.5 |
| Soybean Oil | 13.9 |
| Vinegar, 120 grain | 7.7 |
| Sterol/Corn Starch/PGE 55AK Complex (49:49:2 Ratio) | 6.5 |
| Spices/Flavorings | 4.9 |
| Sugar | 4.2 |
| Garlic Puree in Vinegar | 4.0 |
| Salt | 1.8 |
| Xanthan Gum | 0.4 |
| Potassium Sorbate | 0.05 |
| Vitamin E | 0.006 |
| EDTA | 0.006 |

Water, sugar, salt, vitamin E, EDTA, and potassium sorbate were added to a Hobart bowl according to the formula shown above. The ingredients were then mixed at a first speed until the sugar and salt dissolved. Next, the mixing speed was increased to a second speed and the sterol-OSA starch-PGE 55AK complex in a 49:49:2 ratio was added to the blend according to the formula shown above; mixing continued for an additional 2 minutes. Then, the mixing speed was reduced to the first speed and a slurry of xanthan gum in a part of the oil specified above was added to the mixture. The remaining soybean oil was added. The blend was then mixed for an additional 2 minutes. Then, the vinegar was added followed by mixing for 1 minute. The blend was then homogenized by passing it through a Hydroshear homogenizer. Next, spices and flavorings (including garlic puree) were added according to the above formula and the entire blend was mixed for 1 minute.

The resultant product was similar to the control dressing in taste. Additionally, the dressing from run 3 did not exhibit any gritty and/or grainy mouthfeel generally associated with free sterol. Further testing showed that the percent acid of the product was 1.18%, the pH was 3.54, and viscosity was 7600 cp at room temperature. A single serving of the dressing (about 31g) would provide about 1g sterol. Photomicrographic results showed that PGE 55AK inhibited the growth of sterol crystals.

### Example 5. Incorporation of Sterol-Starch Complex into Light Mayonnaise.

**Control Run.** Production of Light Mayonnaise without Sterol/Starch Complex. As a control, light mayonnaise was produced without the addition of sterol-corn starch complex according to the following formulas listed for a starch base and an emulsion base below:

| **Starch Base Ingredient** | **Amount (%)** |
|---|---|
| Water | 39.0 |
| Modified Starch | 5.4 |
| Lactic Acid (50%) | 0.5 |
| Vinegar, 120 grain | 1.2 |
| Total Starch Base | 46.0 |

| **Emulsion Base Ingredient** | **Amount (%)** |
|---|---|
| Water | 29.8 |
| Soybean Oil | 13.9 |
| Salted Egg Yolk | 6.5 |
| Salt | 1.8 |
| Sugar | 1.5 |
| Xanthan Gum | 0.2 |
| Potassium Sorbate | 0.1 |
| Spices/Flavorings | 0.08 |
| Phosphoric Acid (80%) | 0.04 |
| EDTA | 0.007 |
| Total Emulsion Base | 54.0 |

First, a starch base was prepared by cooking a mixture of water, starch (National 377), lactic acid, and vinegar, in the amounts provided above, at 190°F. Next, an emulsion base was prepared by adding water, salt, potassium sorbate, EDTA, salted egg yolk, spices and flavorings, and sugar to a Hobart bowl according to the formula above. The ingredients were mixed at a first speed for 1 minute. Next, the mixing speed was decreased to a second speed and xanthan gum slurry in a part of the oil specified above was added and mixed for 1 minute. The remaining oil was added according to the amount specified above. Next, phosphoric acid was added and the mixture was stirred for 1 additional minute. The pre-emulsion blend was homogenized in a high shear short time (HSST) mixer. Finally, the final emulsion was mixed with the starch base, as provided above, in a 54:46 ratio in a Hobart Bowl.

As expected, the resultant product exhibited the normal qualities of light mayonnaise.

**Run 1.** Incorporation of 8.0% Sterol-Corn Starch Complex in a 50:50 Ratio. In run 1, light mayonnaise was produced with the addition of 8.0% sterol-corn starch complex at a 50:50 ratio (prepared in a manner similar to that described in Example 3, run 1) according to the formulas listed below:

| **Ingredient** | **Amount (%)** |
|---|---|
| Water | 39.0 |
| Modified Starch | 5.4 |
| Lactic Acid (50%) | 0.5 |
| Vinegar; 120 grain | 1.2 |
| Total Starch Base | 46.0 |

| **Ingredient** | **Amount (%)** |
|---|---|
| Water | 21.5 |
| Soybean Oil | 14.3 |
| Sterol/Corn Starch Complex (50:50 Ratio) | 8.0 |
| Salted Egg Yolk | 6.5 |
| Salt | 1.8 |
| Sugar | 1.5 |
| Xanthan Gum | 0.04 |
| Potassium Sorbate | 0.1 |
| Spices/Flavorings | 0.08 |
| Phosphoric Acid (80%) | 0.04 |
| EDTA | 0.007 |
| Total Emulsion Base | 54.0 |

First, a starch base was prepared by cooking a mixture of water, starch (National 377), lactic acid, and vinegar, in the amounts provided above, at 190°F. Next, an emulsion base was prepared by adding water, salt, potassium sorbate, EDTA, salted egg yolk, spices and flavorings, sugar, and the sterol-corn starch complex (50:50 ratio) to a Hobart bowl according to the formula above. The ingredients were mixed at a first speed for 1 minute. Next, the mixing speed was decreased to a second speed and xanthan gum slurry in a part of the oil specified above was added and mixed for 1 minute. The remaining oil was added according to the amount specified above. Next, phosphoric acid was added and the mixture was stirred for 1 additional minute. The pre-emulsion blend was homogenized in a high shear short time (HSST) mixer. Finally, the final emulsion was mixed with the starch base, as provided above, in a 54:46 ratio in a Hobart Bowl.

The resultant product was similar to the control mayonnaise in taste. Additionally, the mayonnaise from run 1 did not exhibit any gritty and/or grainy mouthfeel generally associated with free sterol although the product contained 0.6g of sterol per serving (about 15g).

**Run 2.** Incorporation of 8.0% Sterol-OSA Starch Complex in a 50:50 Ratio. In run 2, light mayonnaise was produced with the addition of 8.0% sterol-OSA starch complex at a 50:50 ratio (prepared in a manner similar to that described in Example 3 (Run 2)) according to the formulas listed below:

| **Ingredient** | **Amount (%)** |
|---|---|
| Water | 39.0 |
| Modified Starch | 5.4 |
| Lactic Acid (50%) | 0.5 |
| Vinegar, 120 grain | 1.2 |
| Total Starch Base | 46.0 |

| **Ingredient** | **Amount (%)** |
|---|---|
| Water | 21.4 |
| Soybean Oil | 14.3 |
| Sterol/OSA Starch Complex (50:50 Ratio) | 8.0 |
| Salted Egg Yolk | 6.5 |
| Salt | 1.8 |
| Sugar | 1.5 |
| Xanthan Gum | 0.2 |
| Potassium Sorbate | 0.1 |
| Spices/Flavorings | 0.08 |
| Phosphoric Acid (80%) | 0.04 |
| EDTA | 0.007 |
| Total Emulsion Base | 54.0 |

First, a starch base was prepared by cooking a mixture of water, starch (National 377), lactic acid, and vinegar, in the amounts provided above, at 190°F. Next, an emulsion base was prepared by adding water, salt, potassium sorbate, EDTA, salted egg yolk, spices and flavorings, sugar, and the sterol-OSA starch complex (50:50 ratio) to a Hobart bowl according to the formula above. The ingredients were mixed at a first speed for 1 minute. Next, the mixing speed was decreased to a second speed and xanthan gum slurry in a part of the oil specified above was added and mixed for 1 minute. The remaining oil was added according to the amount specified above. Next, phosphoric acid was added and the mixture was stirred for 1 additional minute. The pre-emulsion blend was homogenized in a high shear short time (HSST) mixer. Finally, the final emulsion was mixed with the starch base, as provided above, in a 54:46 ratio in a Hobart Bowl.

The resultant product was similar to the control mayonnaise in taste. Additionally, the mayonnaise from run 2 did not exhibit any gritty and/or grainy mouthfeel generally associated with free sterol although the product contained 0.6g of sterol per serving (about 15g).

**Run 3.** Incorporation of 8.2% Sterol-OSA-PGE 55AK Starch Complex in a 49:49:2 Ratio. In run 3, creamy light mayonnaise was produced with the addition of 8.2% sterol-OSA starch-PGE 55AK complex at a 49:49:2 ratio (prepared in a manner similar to that described in Example 3, run 3) according to the formulas listed below:

| **Ingredient** | **Amount (%)** |
|---|---|
| Water | 39.0 |
| Modified Starch | 5.4 |
| Lactic Acid (50%) | 0.5 |
| Vinegar, 120 grain | 1.2 |
| Total Starch Base | 46.0 |

| **Ingredient** | **Amount (%)** |
|---|---|
| Water | 21.2 |
| Soybean Oil | 14.3 |
| Sterol/OSA Starch/PGE 55AK Complex (49:49:2 Ratio) | 8.2 |
| Salted Egg Yolk | 6.5 |
| Salt | 1.8 |
| Sugar | 1.5 |
| Xanthan Gum | 0.2 |
| Potassium Sorbate | 0.1 |
| Spices/Flavorings | 0.08 |
| Phosphoric Acid (80%) | 0.04 |
| EDTA | 0.007 |
| Total Emulsion Base | 54.0 |

First, a starch base was prepared by cooking a mixture of water, starch (National 377), lactic acid, and vinegar in the amounts provided above, at 190°F. Next, an emulsion base was prepared by adding water, salt, potassium sorbate, EDTA, salted egg yolk, spices and flavorings, sugar, and the sterol-OSA starch-PGE 55AK complex (49:49:2 ratio) to a Hobart bowl according to the formula above. The ingredients were mixed at a first speed for 1 minute. Next, the mixing speed was decreased to a second speed and xanthan gum slurry in a part of the oil specified above was added and mixed for 1 minute. The remaining oil was added according to the amount specified above. Next, phosphoric acid was added and the mixture was stirred for 1 additional minute. The pre-emulsion blend was homogenized in a high shear short time (HSST) mixer. Finally, the final emulsion was mixed with the starch base, as provided above, in a 54:46 ratio in a Hobart Bowl.

The resultant product was similar to the control mayonnaise in taste. Additionally, the mayonnaise from run 3 did not exhibit any gritty and/or grainy mouthfeel generally associated with free sterol although the product contained 0.6g of sterol per serving (about 15 g). Additionally, the addition of the crystal inhibitor PGE 55AK inhibited the growth of sterol crystals.

**Example 6. Incorporation of Sterol-Corn Starch Complex in a Strawberry Drink.** A mixture of sugar, citric acid, sterol-corn starch complex (in a 50:50 ratio as prepared in Example 3, run 1), and xanthan gum, as shown in the formula below was added to water in a Hobart Bowl and mixed at a speed 2 for 2 minutes. Raspberry flavor and puree were then added to the mixture in the Hobart Bowl and further mixed for a minute.

| **Ingredients** | **Amount (g)** |
|---|---|
| Water | 400.2 |
| Sugar | 39.2 |
| Citric Acid | 0.25 |
| Sterol/Corn Starch (50:50) Complex | 10.2 |
| Xanthan Gum | 0.7 |
| Raspberry Flavor | 0.5 |
| Raspberry Puree | 49.0 |

A 240g serving of the strawberry drink provided 2.3g of sterol.

## Claims

1. A method of forming a plant sterol-carbohydrate complex for incorporation in a food product, the method comprising (a) melting a plant sterol and a carbohydrate in the presence of water at a pressure sufficient to prevent the water from boiling off and (b) cooling the mixture to form the plant sterol-carbohydrate complex, wherein the complex is water soluble and can be directly incorporated into the food product in an amount effective to reduce serum cholesterol levels in a human consuming such food product, without adversely modifying the organoleptic properties of the food product.

2. The method of Claim 1, wherein the plant sterol is a sterol selected from the group consisting of β-sitosterol, campesterol, stigmasterol, and ergosterol.

3. The method of Claim 1 or 2, wherein the plant sterol is a stanol selected from the group consisting of β-sitostanol and campestanol.

4. The method of any one of Claims 1 to 3, wherein the carbohydrate is an amylose-containing starch.

5. The method of any one of Claims 1 to 3, wherein the carbohydrate is selected from the group consisting of high-amylose starch, maltodextrin, corn starch, octenyl succinylated starch, and acetylated starch.

6. The method of any one of Claims 1 to 5, wherein the plant sterol-carbohydrate complex includes a crystal growth inhibitor.

7. The method of Claim 6, wherein the crystal growth inhibitor is a polyglycerol ester or a polyglycerol polyricinoleate.

8. The method of any one of Claims 1 to 7, wherein the plant sterol-carbohydrate complex is in a solid form.

9. The method of any one of Claims 1 to 8, wherein the mixture of the plant sterol and the carbohydrate in water are melted at a temperature of about 120 to about 190°C.

10. The method of any one of Claims 1 to 9, wherein the plant sterol-carbohydrate complex is formed using an extruder.

11. A plant sterol-carbohydrate complex having cholesterol-reducing properties when incorporated into a diet of a human, wherein the plant sterol-carbohydrate complex is prepared according to the method of any one of Claims 1 to 10.

12. A food product comprising a cholesterol-reducing amount of a plant sterol, wherein the plant sterol is added to the food product in the form of the plant sterol-carbohydrate complex of Claim 11.

13. A plant sterol-protein complex having cholesterol-reducing properties when incorporated into a diet of a human, wherein the plant sterol-carbohydrate complex is prepared by (a) melting a mixture of a plant sterol and a protein in water at a pressure sufficient to prevent the water from boiling off and (b) cooling the mixture to form the plant sterol-protein complex, wherein the plant sterol-protein complex is water soluble and has cholesterol-reducing properties when incorporated into the diet.

14. The plant sterol-protein Complex of Claim 13 ,wherein the protein is zein, wheat gluten, wheat flour, wheat protein concentrate, soy flour, soy protein concentrate, or soy protein isolate.

15. The plant sterol-protein of Claim 13 or 14, wherein the plant sterol is a sterol selected from the group consisting of β-sitosterol, campesterol, stigmasterol, and ergosterol.

16. The plant sterol-protein complex of Claim 13 or 14, wherein the plant sterol is a stanol selected from the group consisting of β-sitostanol and campestanol.
